# EUROPEAN PATENT APPLICATION

(11) **EP 4 234 236 A1**
(43) Date of publication of application: **30.08.2023**
(21) Application number: 21882922.4
(22) Date of filing: 22.10.2021
(51) Int. Cl.: B32B 27/30, C08F 257/02, C09D 5/02, C09D 5/16, C08F 212/04, C08F 220/26, C09D 133/14, C12M 3/00, C12N 5/02, C09D 7/20

(54) **BIOLOGICAL SUBSTANCE LOW-ADHESION MATERIAL COMPRISING COPOLYMER**

(30) Priority: 23.10.2020 JP 2020178079
(71) Applicant: Nissan Chemical Corporation, Tokyo 103-6119 (JP)
(72) Inventor: HIROI Miya, Funabashi-shi, Chiba 274-0052 (JP); TADOKORO Shinsuke, Funabashi-shi, Chiba 274-8507 (JP); KOZAWA Masami, Funabashi-shi, Chiba 274-8507 (JP); HIROI Yoshiomi, Funabashi-shi, Chiba 274-0052 (JP)
(74) Representative: dompatent von Kreisler Selting Werner - Partnerschaft von Patent- und Rechtsanwälten mbB
(86) International application number: PCT/JP2021/039057
(87) International publication number: WO 2022/085783

(57) **Abstract**

An object of the present invention is to provide a novel composition for forming a coating film having an ability to suppress adhesion of biological substances, a coating film using the same, a substrate for cell culture using the same, a laminated film and a method for producing a cell aggregate(s).

The present invention is to provide a composition for forming a coating film which comprises a polymer containing unit structures represented by the formula (1) and the formula (2): 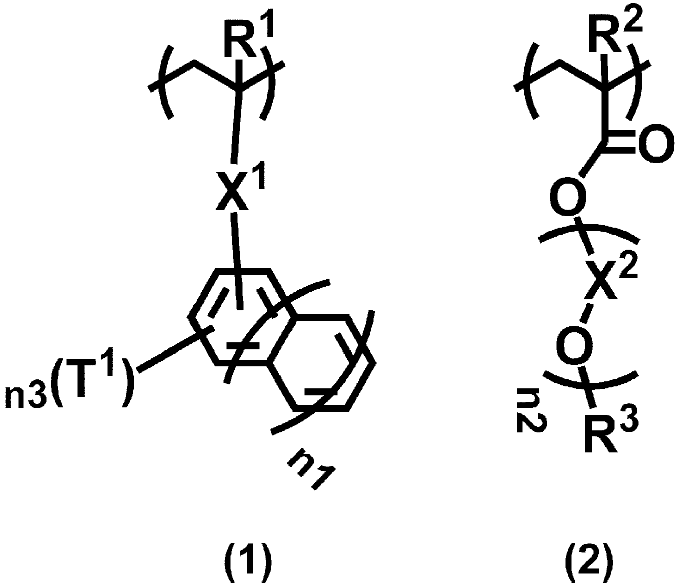 (wherein R¹ to R³, X¹, X², T¹, n1, n2 and n3 are as defined in the present specification and claims) but not containing a polysiloxane skeleton, and a solvent.

## Description

### TECHNICAL FIELD

The present invention relates to a composition for forming a coating film containing a copolymer having a specific structure, preferably a composition for forming a coating film having an ability to suppress adhesion of biological substances, a coating film using the same, a substrate for cell culture using the same, a laminated film and a method for producing a cell aggregate(s).

### BACKGROUND ART

Various coating materials having an ability to suppress adhesion of biological substances have been proposed for suppressing adhesion of biological substances to devices for medical use such as artificial dialyzers, artificial organs, medical devices, etc., and various kinds of devices for medical use such as cell culture containers, substrates for cell culture, etc.

In Patent Document 1, there is disclosed an ion complex material having an ability to suppress adhesion of a biological substance. In Patent Document 2, there are disclosed a method for producing a polymer used as a base film for cell culture and a cell culture container.

### PRIOR ART DOCUMENTS

### PATENT DOCUMENTS

Patent Document 1: WO 2014/196650
Patent Document 2: WO 2020/040247

### SUMMARY OF THE INVENTION

### PROBLEMS TO BE SOLVED BY THE INVENTION

The object of the present invention is to provide a novel composition for forming a coating film for forming a coating film having an ability to suppress adhesion of biological substances, a coating film using the same, a substrate for cell culture using the same, a laminated film and a method for producing a cell aggregate(s).

### MEANS TO SOLVE THE PROBLEMS

The present invention includes the following.
[1] A composition for forming a coating film which comprises a copolymer (P¹) containing unit structures represented by the formula (1) and the formula (2): 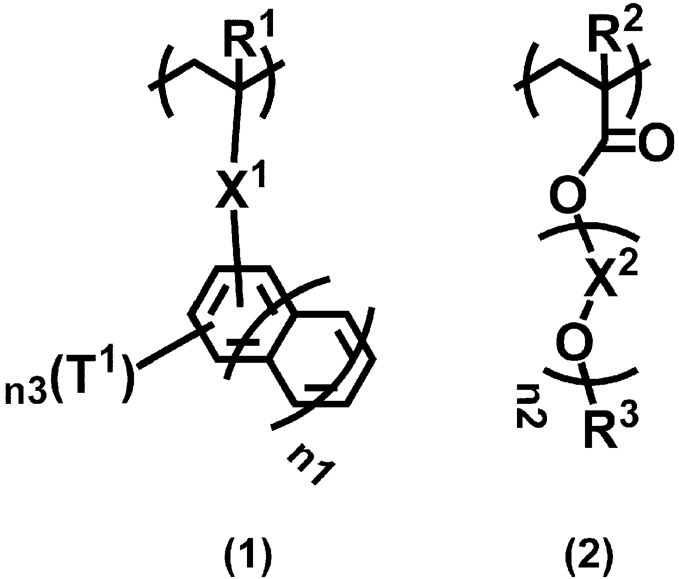 (wherein R¹ to R³ each independently represent a hydrogen atom or an alkyl group having 1 to 5 carbon atoms, X¹ represents a single bond, an ester bond, an amide bond or an alkylene group having 1 to 5 carbon atoms, X² represents an alkylene group having 1 to 5 carbon atoms, T¹ represents a halogen atom, an alkyl group having 1 to 5 carbon atoms which may be substituted by a halogen atom(s), a hydroxyl group, a carboxyl group, a nitro group, a cyano group, a methylenedioxy group, an acetoxy group, a methylthio group, an amino group or an alkoxy group having 1 to 10 carbon atoms, n1 represents an integer of 0 to 2, n2 represents an integer of 1 to 10 and n3 represents an integer of 0 to 8), but not containing a polysiloxane skeleton, and a solvent.
[2] The composition described in [1], wherein the solvent is selected from water, an alcohol or a combination thereof.
[3] The composition for forming a coating film described in [1] or [2], which is in a form of a colloidal solution containing the solvent as a dispersion medium, and containing the copolymer (P¹) as a dispersoid.
[4] The composition for forming a coating film described in any one of [1] to [3], which has an ability to suppress adhesion of biological substances.
[5] A coating film which comprises a copolymer (P¹) containing unit structures represented by the formula (1) and the formula (2): 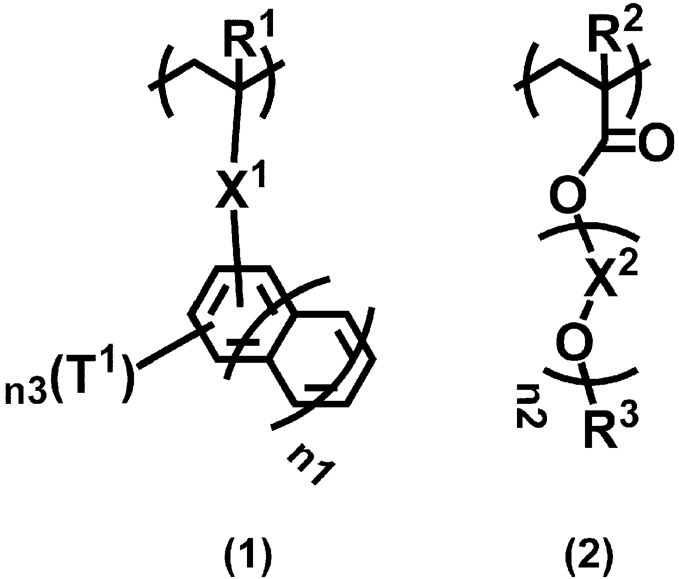 (wherein R¹ to R³ each independently represent a hydrogen atom or an alkyl group having 1 to 5 carbon atoms, X¹ represents a single bond, an ester bond, an amide bond or an alkylene group having 1 to 5 carbon atoms, X² represents an alkylene group having 1 to 5 carbon atoms, T¹ represents a halogen atom, an alkyl group having 1 to 5 carbon atoms which may be substituted by a halogen atom(s), a hydroxyl group, a carboxyl group, a nitro group, a cyano group, a methylenedioxy group, an acetoxy group, a methylthio group, an amino group or an alkoxy group having 1 to 10 carbon atoms, n1 represents an integer of 0 to 2, n2 represents an integer of 1 to 10 and n3 represents an integer of 0 to 8), but not containing a polysiloxane skeleton.
[6] The coating film described in [5], which has an ability to suppress adhesion of biological substances.
[7] A substrate for cell culture which comprising having the coating film described in [6] at least part of a surface of the substrate.
[8] A laminated film which comprises a layer of the coating film described in [5] or [6], and a layer of a base film for cell culture on the upper surface thereof
[9] The laminated film described in [8], wherein the base film for cell culture contains a copolymer (P²) which contains a recurring unit derived from a monomer represented by the following formula (I): 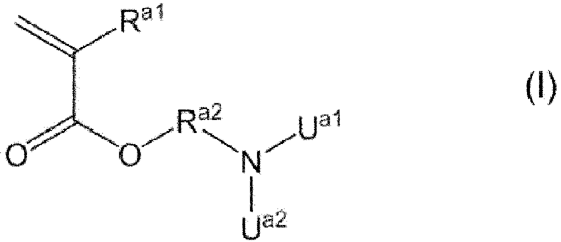 [wherein
   U^{a1} and U^{a2} each independently represent a hydrogen atom or an alkyl group having 1 to 5 carbon atoms, R^{a1} represents a hydrogen atom or an alkyl group having 1 to 5 carbon atoms, and R^{a2} represents an alkylene group having 1 to 5 carbon atoms], and a recurring unit derived from a monomer represented by the following formula (II): 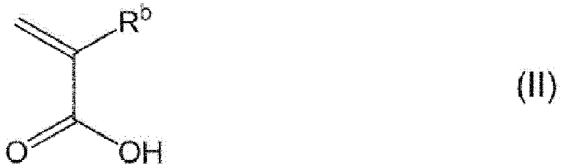 [wherein
   R^{b} represents a hydrogen atom or an alkyl group having 1 to 5 carbon atoms].
[10] A substrate for cell culture provided with the laminated film described in [8] or [9].
[11] A method for producing a cell aggregate(s), which comprises a step of seeding cells to the substrate for cell culture described in [7] or [10].
[12] A coating film which is obtained by washing a coating film which contains a copolymer (P¹) containing unit structures represented by the formula (1) and the formula (2): 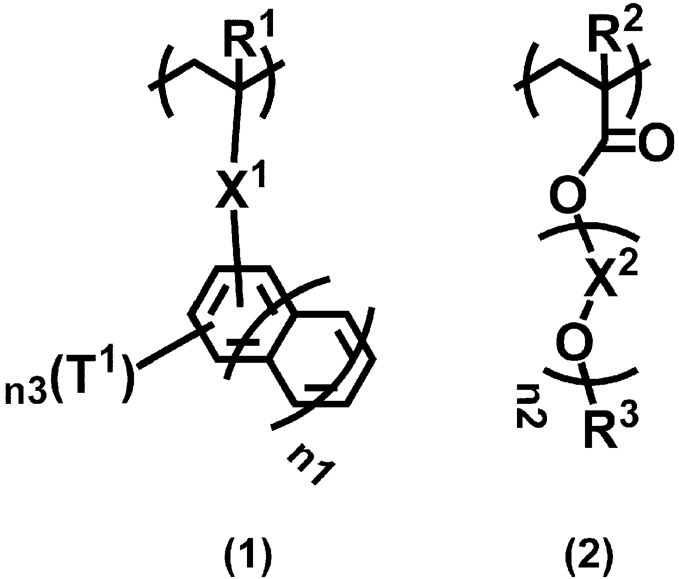 (wherein R¹ to R³ each independently represent a hydrogen atom or an alkyl group having 1 to 5 carbon atoms, X¹ represents a single bond, an ester bond, an amide bond or an alkylene group having 1 to 5 carbon atoms, X² represents an alkylene group having 1 to 5 carbon atoms, T¹ represents a halogen atom, an alkyl group having 1 to 5 carbon atoms which may be substituted by a halogen atom(s), a hydroxyl group, a carboxyl group, a nitro group, a cyano group, a methylenedioxy group, an acetoxy group, a methylthio group, an amino group or an alkoxy group having 1 to 10 carbon atoms, n1 represents an integer of 0 to 2, n2 represents an integer of 1 to 10 and n3 represents an integer of 0 to 8), but not containing a polysiloxane skeleton, with a solvent.
[13] A method for producing a coating film which comprises a step of washing a coating film which contains a polymer containing unit structures represented by the formula (1) and the formula (2): 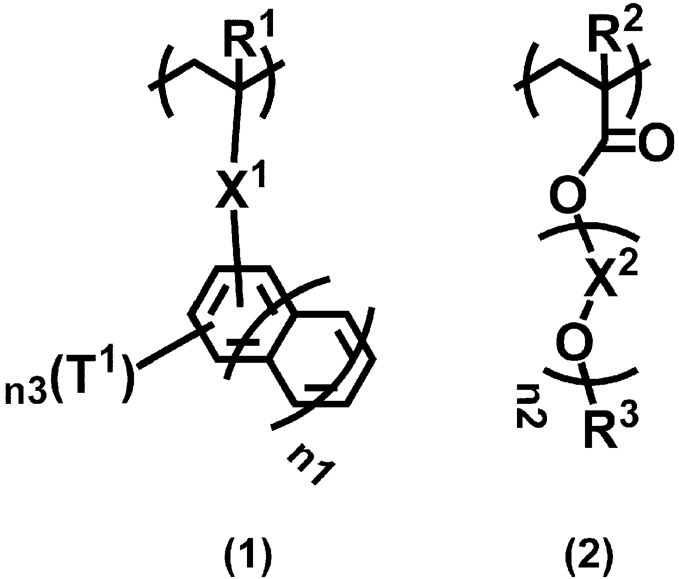 (wherein R¹ to R³ each independently represent a hydrogen atom or an alkyl group having 1 to 5 carbon atoms, X¹ represents a single bond, an ester bond, an amide bond or an alkylene group having 1 to 5 carbon atom, X² represents an alkylene group having 1 to 5 carbon atom, T¹ represents a halogen atom, an alkyl group having 1 to 5 carbon atoms which may be substituted by a halogen atom(s), a hydroxyl group, a carboxyl group, a nitro group, a cyano group, a methylenedioxy group, an acetoxy group, a methylthio group, an amino group or an alkoxy group having 1 to 10 carbon atoms, n1 represents an integer of 0 to 2, n2 represents an integer of 1 to 10 and n3 represents an integer of 0 to 8), but not containing a polysiloxane skeleton, with a solvent.

### EFFECTS OF THE INVENTION

As a result of earnest studies by the inventors of the present application, when a random copolymer or a block copolymer containing unit structures represented by the formula (1) and the formula (2) is synthesized and formed a coating film on a substrate, then, it is found that they have excellent ability to suppress adhesion of cells or protein, whereby the present invention has been accomplished. It is presumed that by taking a structure that the hydrophobic portion of the unit structure represented by the formula (1) adheres to the substrate and the hydrophilic portion of the unit structure represented by the formula (2) is exposed on the surface, the surface of the substrate exhibits hydrophilic property and it has specific characteristics to biological substances.

### BRIEF DESCRIPTION OF THE DRAWINGS

Fig. 1 is a schematic drawing of a flow reactor (reaction device) used in Synthetic Examples.
Fig. 2(a) and (b) are ¹H-NMR charts of the polymer obtained in Synthetic Example 1.
Fig. 3 is observation results of cell adhesion in Test Example 4.
Fig. 4 is observation results of cell adhesion in Test Example 5.
Fig. 5 is observation results of cell adhesion in Test Example 6.
Fig. 6 is observation results of cell aggregates of Test Example 6.
Fig. 7 is observation results of cell adhesion in Test Example 7.
Fig. 8 is observation results of cell aggregates of Test Example 7.

### EMBODIMENTS TO CARRY OUT THE INVENTION

### <Composition for forming coating film>

The composition for forming a coating film of the present invention contains a copolymer (P¹) which contains unit structures represented by the formula (1) and the formula (2): 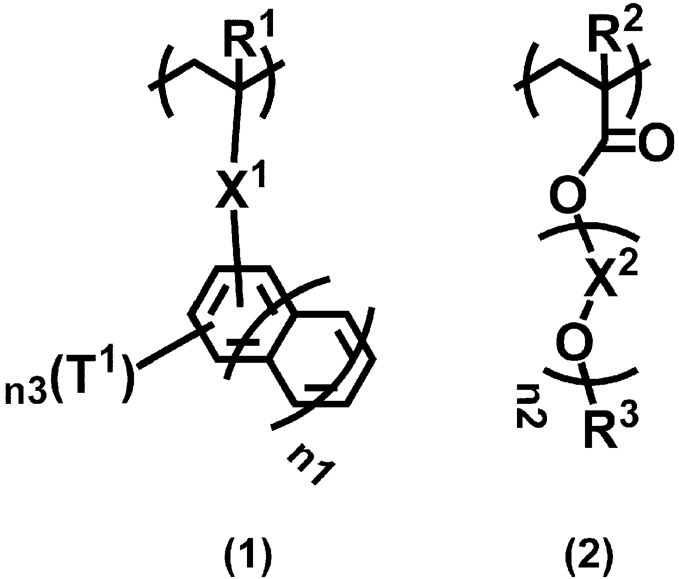 (wherein R¹ to R³ each independently represent a hydrogen atom or an alkyl group having 1 to 5 carbon atoms, X¹ represents a single bond, an ester bond, an amide bond or an alkylene group having 1 to 5 carbon atoms, X² represents an alkylene group having 1 to 5 carbon atoms, T¹ represents a halogen atom, an alkyl group having 1 to 5 carbon atoms which may be substituted by a halogen atom(s), a hydroxyl group, a carboxyl group, a nitro group, a cyano group, a methylenedioxy group, an acetoxy group, a methylthio group, an amino group or an alkoxy group having 1 to 10 carbon atoms, n1 represents an integer of 0 to 2, n2 represents an integer of 1 to 10 and n3 represents an integer of 0 to 8), and a solvent.

As the above-mentioned "alkyl group having 1 to 5 carbon atoms", there may be mentioned a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group. R¹ to R³ each independently and preferably selected from a hydrogen atom, a methyl group or an ethyl group.

The above-mentioned "ester bond" means -C(=O)-O- or -O-C(=O)-, and the "amide bond" means -NHC(=O)- or -C(=O)NH-.

As the above-mentioned "alkylene group having 1 to 5 carbon atoms", there may be mentioned a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group and a 1-ethyl-trimethylene group. As the above-mentioned X¹ and X², they are preferably selected from a methylene group, an ethylene group and a propylene group.

As the above-mentioned "halogen atom", there may be mentioned a fluorine atom, a chlorine atom, a bromine atom and an iodine atom.

The above-mentioned "alkyl group having 1 to 5 carbon atoms which may be substituted by a halogen atom(s)" means an alkyl group having 1 to 5 carbon atoms, or the above-mentioned alkyl group having 1 to 5 carbon atoms substituted by one or more above-mentioned halogen atom(s). Examples of the "alkyl group having 1 to 5 carbon atoms" are as mentioned above. The "alkyl group having 1 to 5 carbon atoms substituted by one or more halogen atom(s)" means one or more optional hydrogen atoms of the above-mentioned alkyl group having 1 to 5 carbon atoms is/are substituted by the halogen atom(s), and examples may be mentioned a fluoromethyl group, a difluoromethyl group, a trifluoromethyl group, a chloromethyl group, a dichloromethyl group, a trichloromethyl group, a bromomethyl group, an iodomethyl group, a 2,2,2-trifluoroethyl group, a 2,2,2-trichloroethyl group, a perfluoroethyl group, a perfluorobutyl group or a perfluoropentyl group, etc.

As the above-mentioned "alkoxy group having 1 to 10 carbon atoms", there may be mentioned a methoxy group, an ethoxy group, an n-propoxy group, an i-propoxy group, an n-butoxy group, an i-butoxy group, an s-butoxy group, a t-butoxy group, an n-pentoxy group, a 1-methyl-n-butoxy group, a 2-methyl-n-butoxy group, a 3-methyl-n-butoxy group, a 1,1-dimethyl-n-propoxy group, a 1,2-dimethyl-n-propoxy group, a 2,2-dimethyl-n-propoxy group, a 1-ethyl-n-propoxy group, an n-hexyloxy group, a 1-methyl-n-pentyloxy group, a 2-methyl-n-pentyloxy group, a 3-methyl-n-pentyloxy group, a 4-methyl-n-pentyloxy group, a 1,1-dimethyl-n-butoxy group, a 1,2-dimethyl-n-butoxy group, a 1,3-dimethyl-n-butoxy group, a 2,2-dimethyl-n-butoxy group, a 2,3-dimethyl-n-butoxy group, a 3,3-dimethyl-n-butoxy group, a 1-ethyl-n-butoxy group, a 2-ethyl-n-butoxy group, a 1,1,2-trimethyl-n-propoxy group, a 1,2,2,-trimethyl-n-propoxy group, a 1-ethyl-1-methyl-n-propoxy group, a 1-ethyl-2-methyl-n-propoxy group, an n-heptyloxy group, an n-octyloxy group and an n-nonyloxy group.

The unit structures represented by the above-mentioned formula (1) and formula (2) are preferably derived from compounds represented by the following formula (1-1) and the formula (2-1): 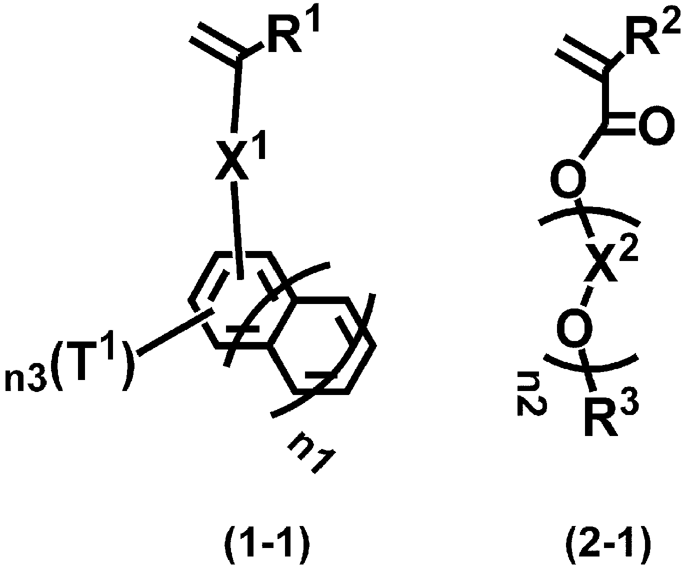 (R¹ to R³, X¹, X², T¹, n1, n2 and n3 are the same as defined above). Incidentally, when these compounds contain a functional group(s), the compounds wherein the functional group(s) is/are suitably protected by the known protective group(s) are also similarly preferable.

In the monomer represented by the formula (1-1), R¹ preferably represents a hydrogen atom or a methyl group, and X¹ preferably represents a single bond, an ester bond, an amide bond or a methylene group. Specific examples of such a monomer may be mentioned styrene, vinylnaphthalene and vinyl anthracene. The aryl group in the above-mentioned monomer may be substituted by the above-mentioned T¹. Among these, unsubstituted styrene is preferable.

In the monomer represented by the formula (2-1), R² and R³ preferably, each independently represent a hydrogen atom or a methyl group, X² preferably represents an ethylene group or a propylene group, and n2 represents an integer of 1 to 5. Specific examples of such a monomer may be mentioned ethylene glycol mono(meth)acrylate (=2-hydroxyethyl (meth)acrylate), ethylene glycol monomethyl ether (meth)acrylate, diethylene glycol mono(meth)acrylate, diethylene glycol monomethyl ether (meth)acrylate, triethylene glycol mono(meth)acrylate, triethylene glycol monomethyl ether (meth)acrylate, dipropylene glycol mono(meth)acrylate, dipropylene glycol monomethyl ether (meth)acrylate, or a monomer in which the hydroxy group thereof is protected depending on necessity, etc., and it is preferable to use ethylene glycol monomethacrylate, diethylene glycol monomethyl ether methacrylate or triethylene glycol monomethyl ether methacrylate.

Incidentally, in the present invention, the (meth)acrylate compound means an acrylate compound or a methacrylate compound. For example, (meth)acrylate means acrylate or methacrylate.

The copolymer (P¹) of the present application may be either of a random copolymer or a block copolymer. The copolymer (P¹) of the present application can be produced by polymerizing the monomers of the above-mentioned formula (1-1) and the formula (2-1) by a conventionally known method. The copolymer (P¹) of the present application is preferably derived from each one kind of the compounds which derives the above-mentioned formula (1) and the formula (2), but each two or more kinds of compounds may be derived.

A compound having a recurring unit of the third component represented by the formula other than the above-mentioned formula (1) and the formula (2) may be further polymerized.

A molar ratio of each unit structure represented by the formula (1) and the formula (2) is, for example, the formula (1) : the formula (2) = 1 to 90 : 99 to 10, preferably 1 to 70 : 99 to 30, more preferably 1 to 50 : 99 to 50, further preferably 1 to 30 : 99 to 70, and particularly preferably 2 to 25:98 to 75.

A molar ratio of the unit structures represented by the formula (1) and the formula (2) based on the whole copolymer (P¹) is not particularly limited as long as the effects of the present application can be accomplished and, for example, it is 80 mol% or more, 90 mol% or more, and 100 mol%.

It is preferable that the copolymer (P¹) of the present application does not contain a polysiloxane skeleton. The polysiloxane skeleton means a skeleton containing a repetition of a siloxane unit structure: -OSiRR- (R is a hydrogen, an alkyl group, etc.).

The above-mentioned block copolymer can be synthesized by the conventionally known method and, for example, it may be a material produced by using a mixer described in WO 2017/135398. The entire disclosure described in WO 2017/135398 is incorporated in the present application by reference.

The block copolymer of the present application is a material in which a plural kinds of polymers (block) that are chemically different and covalently bonded to each other are bound. The block copolymer to be used in the invention of the present application contains an organic polymer chain (A) which contains an organic monomer (a) as a unit structure, and a polymer chain (B) which contains a monomer (b) different from the organic monomer (a) as a unit structure and binds to the organic polymer chain (A).

A solid content of the composition for forming a coating film of the present application can be made 0.01 to 50% by mass, or 0.1 to 20% by mass, or 0.1 to 10% by mass. The solid content is the remaining ratio of the composition for forming a film from which the solvent has been removed.

A ratio of the block copolymer occupied in the solid content can be made 30 to 100% by mass, or 50 to 100% by mass, or 50 to 90% by mass, or 50 to 80% by mass.

The kind of the blocks existing in the block copolymer can be made 2 or 3 kinds or more. And, a number of the blocks existing in the block copolymer can be made 2 or 3 or more.

By changing the polymer chain (B), it is possible to use, for example, an adjacent polymer chain (C) containing the monomer (c) as a unit structure. As the block polymer, there are combinations of AB, ABAB, ABA, ABC, etc.

As one of the methods for synthesizing a block copolymer, it can be obtained by living radical polymerization, living cationic polymerization or living anionic polymerization in which the polymerization process consists only of an initiation reaction and a growth reaction and is not accompanied by a side reaction that inactivates the growth end. The growth end can continue to maintain the growth activity reaction during the polymerization reaction. By inhibiting to cause chain transfer, a polymer (A) having a uniform length can be obtained. By utilizing the growth end of this polymer (A), and by adding a different monomer (b), polymerization can proceed under this monomer (b) to form a block copolymer (AB).

Homopolymer A or B is a polymerizable compound having at least one polymerizable reactive group, preferably a radically polymerizable reactive group (a vinyl group or a vinyl group-containing organic group).

A weight average molecular weight Mw of the copolymer (P¹) to be used in the invention of the present application is preferably 1,000 to 1,000,000, 5,000 to 500,000, 5,000 to 100,000 or 5,000 to 50,000. If it is less than 1,000, there is a case where coatability to the base substrate may be poor, while if it exceeds 1,000,000, there is a case where solubility in a solvent may be poor.

A polydispersity (Mw/Mn) of the copolymer (P¹) of the present application is preferably1.00 to 2.50, and particularly preferably 1.00 to 1.50.

### <Solvent>

The solvent contained in the composition for forming a coating film of the present application may be mentioned water, phosphate buffered physiological saline (PBS), and alcohol. As the alcohol, there may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (neopentyl alcohol), 2-methyl-1-propanol, 2-methyl-1-butanol, 2-methyl-2-butanol (t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol. Water, phosphate buffered physiological saline (PBS) or an alcohol may be used alone or a mixed solvent thereof may be used, and from the viewpoint of dissolution of the copolymer (P¹), it is preferable to be selected from water, an alcohol or a combination thereof, and it is more preferable to be selected from water, ethanol or a combination thereof.

### <Colloidal solution (sol)>

The copolymer (P¹) of the present application may become a colloidal solution (sol) in which the solvent becomes a dispersion medium and the above-mentioned copolymer (P¹) becomes a dispersoid depending on the kind of the above-mentioned solvent.

A particle size of the above-mentioned dispersoid is, for example, in the range of 5 to 1,000 nm, preferably 5 to 500 nm, more preferably 5 to 300 nm. The above-mentioned particle size is a value of either the primary particle size or the secondary particle size.

In this case, it is preferable that the solvent is a mixed solvent of water and alcohol. It is preferably a mixed solvent of water and ethanol. A mixing ratio (a weight ratio) of water : alcohol is, for example, 1 to 70 : 99 to 30, and 1 to 50 : 99 to 50.

The composition for forming a coating film of the present invention is preferably a composition for forming a coating film having an ability to suppress adhesion of biological substances.

In the present invention, as the biological substance, there may be mentioned a protein, a saccharide, a virus, a nucleic acid and a cell or a combination thereof, or biological tissues and body fluids containing them.
as the above-mentioned protein, there may be mentioned fibrinogen, bovine serum albumin (BSA), human albumin, various kinds of globulins, β-lipoprotein, various kinds of antibodies (IgG, IgA and IgM), peroxidase, various kinds of complements, various kinds of lectins, fibronectin, lysozyme, von Willebrand factor (vWF), serum γ-globulin, pepsin, ovalbumin, insulin, histone, ribonuclease, collagen and cytochrome c,
as the above-mentioned saccharide, glucose, galactose, mannose, fructose, heparin and hyaluronic acid,
as the above-mentioned nucleic acid, deoxyribonucleic acid (DNA) and ribonucleic acid (RNA), and
as the above-mentioned cells, fibroblast, bone marrow cells, B lymphocytes, T lymphocytes, neutrophils, red blood cells, platelets, macrophages, monocytes, bone cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocytes, microglia, astroglial cells, heart cells, esophagus cells, muscle cells (for example, smooth muscle cells or skeletal muscle cells), pancreatic beta cells, melanocytes, hematopoietic precursor cells, mononuclear cells, embryonic stem cells (ES cell), embryonic tumor cells, embryonic germline stem cells, induced pluripotent stem cells (iPS cell), neural stem cells, hematopoietic stem cells, mesenchymal stem cells, liver stem cells, pancreatic stem cells, muscle stem cells, germline stem cells, intestinal stem cells, cancer stem cells, hair follicle stem cells and various kinds of cell lines (for example, HCT116, Huh7, HEK293 (human embryonic kidney cell), HeLa (human cervical cancer cell lines), HepG2 (human liver cancer cell lines), UT7/TPO (human leukemia cell lines), CHO (Chinese hamster ovary cell lines), MDCK, MDBK, BHK, C-33A, HT-29, AE-1, 3D9, Ns0/1, Jurkat, NIH3T3, PC12, S2, Sf9, Sf21, High Five and Vero), etc.

Also, the above-mentioned antibody may be an antibody drug. The antibody drug is a drug utilizing an antibody, and the antibody is a protein comprising immunoglobulin.

It is preferable that the above-mentioned antibody drug contains at least one of an antibody and an antigen-binding fragment thereof.

It is preferable that the above-mentioned antibody drug contains at least one selected from the group consisting of chimeric antibody, antibodies, human antibodies, humanized antibodies and domain antibodies thereof.

Specific examples of the above-mentioned antibody drug may be mentioned Ofatumumab (product name "Arzerra (Registered trademark)"), Cetuximab (product name "Erbitux (Registered trademark)"), Tocilizumab (product name "Actemra (Registered trademark)"), Bevacizumab (product name "Avastin (Registered trademark)"), Canakinumab (product name "Ilaris (Registered trademark)"), Golimumab (product name "Simponi (Registered trademark)"), Ustekinumab (product name "Stelara (Registered trademark)"), Eculizumab (product name "Soliris (Registered trademark)"), Omalizumab (product name "Xolair (Registered trademark)"), Trastuzumab (product name "Herceptin (Registered trademark)"), Pertuzumab (product name "Perjeta (Registered trademark)"), Adalimumab (product name "Humira (Registered trademark)"), Denosumab (product name "Pralia (Registered trademark)"), product name "Ranmark (Registered trademark)"), Mogamulizumab (product name "Poteligeo (Registered trademark)"), Rituximab (product name "Rituxan (Registered trademark)"), Ranibizumab (product name "Lucentis (Registered trademark)"), Infliximab (product name "Remicade (Registered trademark)"), Aflibercept (product name "Eylea (Registered trademark)"), Abatacept (product name "Orencia (Registered trademark)"), Etanercept (product name "Enbrel (Registered trademark)"), Gemtuzumab ozogamicin (product name "Mylotarg (Registered trademark)"), Panitumumab (product name "Vectibix (Registered trademark)"), Basiliximab (product name "Simulect (Registered trademark)"), Certolizumab pegol (product name "Cimzia (Registered trademark)"), Palivizumab (product name "Synagis (Registered trademark)"), Casirivimab/Imdevimab (product name "Ronapreve (Registered trademark)") and Sotrovimab (product name "Xevudy").

Among these, it is preferable to contain an antibody obtained from a clone derived from a single antibody-producing cell, or a monoclonal antibody which is an antibody molecule, and it is preferably a monoclonal antibody comprising an antihuman CD20 human·antibody and is preferable to contain Rituximab or Abatacept.

Incidentally, the antibody drug may contain one kind of antibody or may contain two or more kinds of antibodies. That is, the antibody drug of the present invention may contain both an antibody and an antigen-binding fragment, may contain two or more kinds of antibodies, and may contain two or more kinds of antigen-binding fragments.

Having an ability to suppress adhesion of protein means that a relative average absorbance (%) ((average absorbance of Example)/(average absorbance with no coating film)) when compared with no coating film in the IgG antibody HRP measurement carried out by the method described in Example is 50% or less, preferably 30% or less, and further preferably 20% or less; and
having an ability to suppress adhesion of cells means that no adhesion nor spreading of cells is observed in the object by microscopic observation, and a cell aggregate(s) (spheroid) is formed.

The above-mentioned biological substance is preferably cells and proteins.

When the above-mentioned biological substance is a cell, the copolymer (P¹) of the present application is preferably the above-mentioned block copolymer.

### <Coating film>

The coating film of the present invention is a coating film which comprises a copolymer (P¹) containing unit structures represented by the formula (1) and the formula (2): 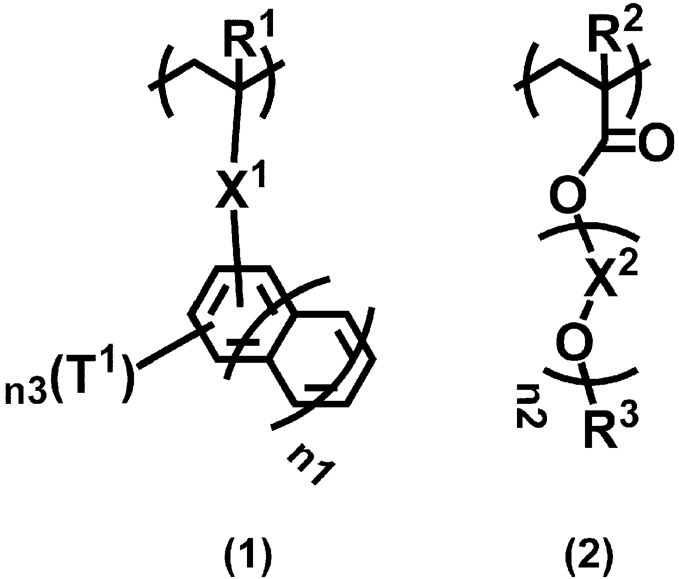 (wherein R¹ to R³ each independently represent a hydrogen atom or an alkyl group having 1 to 5 carbon atoms, X¹ represents a single bond, an ester bond, an amide bond or an alkylene group having 1 to 5 carbon atoms, X² represents an alkylene group having 1 to 5 carbon atoms, T¹ represents a halogen atom, an alkyl group having 1 to 5 carbon atoms which may be substituted by a halogen atom(s), a hydroxyl group, a carboxyl group, a nitro group, a cyano group, a methylenedioxy group, an acetoxy group, a methylthio group, an amino group or an alkoxy group having 1 to 10 carbon atoms, n1 represents an integer of 0 to 2, n2 represents an integer of 1 to 10 and n3 represents an integer of 0 to 8.).

The coating film of the present invention is a coating film of the above-mentioned composition for forming a coating film. The composition for forming a coating film according to the present invention is coated onto a base substance and dried to form a coating film.

As the base substance for forming the coating film of the present invention, the same ones described in the following substrate for cell culture can be used.

In order to form the coating film of the present invention, the above-mentioned composition for forming a coating film is coated onto at least part of the surface of the base substance. The coating method is not particularly limited, and a coating method such as a usual spin coating, dip coating, solvent casting, etc., is used.

A drying step of the coating film according to the present invention is carried out under an atmosphere or vacuum at a temperature in the range of -200°C to 200°C. The coating film can be formed by drying at, for example, room temperature (10°C to 35°C, for example, 25°C), but in order to form the coating film more quickly, it may be dried at, for example, 40°C to 100°C.
A more preferable drying temperature is 10°C to 180°C, and a more preferable drying temperature is 20°C to 150°C.

The coating film of the present application is preferably a coating film having an ability to suppress adhesion of biological substances. It is preferable that the above-mentioned biological substance is a protein and a cell.

When the above-mentioned biological substance is a cell, the copolymer (P¹) contained in the coating film of the present application is preferably the above-mentioned block copolymer.

The coating film of the present application may be a coating film obtained by forming the coating film by the above-mentioned step, and then, further washing.

The above-mentioned washing may be carried out by the known method, and running water washing or ultrasonic wave washing, etc., is desirable. As the washing solvent, there may be mentioned water, and an aqueous solution containing an electrolyte(s). The washing solvent is generally used at room temperature (for example, 10 to 35°C) and may be heated in the range of, for example, 40°C to 95°C. The aqueous solution containing an electrolyte(s) is preferably PBS, physiological saline (containing only sodium chloride), Dulbecco's phosphate buffered physiological saline, tris buffered physiological saline, HEPES buffered physiological saline and Veronal buffered physiological saline, and PBS is particularly preferable. After fixing, the coating film does not dissolve out and firmly fixed to the base substance even if it is washed with water, PBS and alcohol, etc. In particular, when the copolymer (P¹) contained in the above-mentioned coating film is a block copolymer, when it is washed with water or phosphate buffered physiological saline (PBS), it has the effect that change in the film thickness is a little before and after washing, that is, dissolution of the coating film in the solvent is a little.

### <Substrate, substrate for cell culture, and method for producing substrate for cell culture>

The substrate referred to in the present invention may be, in addition to a flat plate substrate having a flat surface, a container or an instrument having an arbitrary structure. Examples of such a substrate may be mentioned instruments for collecting or delivering the above-mentioned biological substance (for example, blood glucose level measuring instrument, injection needles, catheters, etc.), containers for storing the above-mentioned biological substance (for example, bags, bottles, vials, etc., specifically, blood bags, storage containers for antibody drug, etc.), instruments for separating, isolating or analyzing the above-mentioned biological substance (for example, microscope peripheral instruments such as carriers, cover glasses, etc., microchannel derives including flow cytometers such as cell sorters, etc., cuvettes, substrates for cell culture, cell spheroid arrays, cell separation columns, microchannel chips, microwell array chips, assay chips, biochips, magnetic beads, measurement cells for fully automated analyzers, etc.), instruments for biotreatment (for example, reaction tanks, transfer tubes, transfer pipes, instruments for purification, cell culture plates, etc.), prostheses (for example, implants, bone fixation materials, sutures, anti-adhesion membranes, artificial blood vessels, etc., as well as drug delivery media such as vesicles, microparticles and nanoparticles, etc., materials for diagnostic equipment such as gastrocameras, etc., and medical use materials such as microfibers, nanofibers and magnetic particles, etc.

By coating the above-mentioned composition for forming a coating film onto the surface of the substrate and drying the same, a substrate having an ability to suppress adhesion of biological substances can be produced. Here, the "surface" refers to a surface in contact with a biological substance.

The substrate for cell culture of the present invention is a substrate for cell culture having the above-mentioned coating film at least part of the surface of the substrate on the substrate. It is preferable that the above-mentioned coating film is formed over the entire surface on which the cell culture is carried out.

In particular, as the substrate for cell culture, there may be mentioned, for example, dishes (schale) such as petri dishes generally used for cell culture, dishes for cell culture, flasks such as cell culture flasks, spinner flasks, etc., bags such as plastic bags, Teflon (Registered trademark) bags, culture bags, etc., plates such as microplates, microwell plates, multiplates, multiwell plates, etc., chamber slides, tubes, trays, bottles such as roller bottles, etc., a culture vessel having a stirring blade for stirring cell suspension inside, a large culture tank, a bioreactor, etc. There may be preferably mentioned dishes, plates and trays.

Also, the material of the substrate may be mentioned, for example, glass, metal, a metal-containing compound or a semi metal-containing compound, activated charcoal or a resin. The metals may be mentioned typical metals: (alkali metals: Li, Na, K, Rb and Cs; alkaline earth metals: Ca, Sr, Ba and Ra), magnesium group elements: Be, Mg, Zn, Cd and Hg; aluminum group elements: Al, Ga and In; rare earth elements: Y, La, Ce, Pr, Nd, Sm and Eu; tin group elements: Ti, Zr, Sn, Hf, Pb and Th; iron group elements: Fe, Co and Ni; earth acid elements: V, Nb and Ta, chromium group elements: Cr, Mo, W and U; manganese group elements: Mn and Re; noble metals: Cu, Ag and Au; platinum group elements: Ru, Rh, Pd, Os, Ir and Pt, etc. The metal-containing compound or the semi metal-containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a derivative thereof, or a synthetic resin, as the natural resin, there may be mentioned cellulose, cellulose triacetate (CTA), nitrocellulose (NC), cellulose immobilized with dextran sulfate, etc., and as the synthetic resin, there may be preferably used polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), polyvinyl chloride (PVC), polytetrafluoroethylene (PTFE), ultra-high molecular weight polyethylene (UHPE), polydimethylsiloxane (PDMS), acrylonitrile-butadiene-styrene resin (ABS) or Teflon (Registered Trademark). In the production of the substrate for cell culture of the present invention, it does not require a treatment at a high temperature at the time of coating the composition for forming a coating film so as to exist at least part of the surface of the substrate, so that a resin having low heat resistance, etc., can be also applicable.

A material of the substrate may be one kind or a combination of two or more kinds. Among these materials, it is preferably glass, silicon, silicon oxide, polystyrene(PS), polypropylene(PP), polyether sulfone (PES), polyethylene terephthalate (PET), poly carbonate (PC), poly vinyl chloride (PVC), Teflon (Registered Trademark), cycloolefin polymer (COP), polydimethylsiloxane (PDMS) or stainless (SUS304, SUS316, SUS316L, etc.) alone, or a combination selected from these, and particularly preferably glass, polystyrene(PS), polypropylene(PP), stainless (SUS304, SUS316, SUS316L, etc.) or polydimethylsiloxane (PDMS).

As a method for coating the composition for forming a coating film of the present invention, there may be employed, for example, a spin coating method, an inkjet method, a screen printing method, a slit coating method, a roll-to-roll method, etc., and preferably carried out by a printing technique such as the inkjet method or the screen printing method, etc.

As another coating method, for example, there may be employed a method in which the container is immersed in the above-mentioned composition for forming a coating film, the composition for forming a coating film is added to the container and allowed to stand for a predetermined time, or the composition for forming a coating film is coated onto the surface of the container or the substrate, etc., and in the container, as one embodiment, in the case of cell culture containers, it is carried out by the method in which the composition for forming a coating film is added to the container and allowed to stand for a predetermined time. Addition can be carried out by adding, for example, 0.5 to 1-fold amount of the composition for forming a coating film based on the whole volume of the container using a syringe, etc. Allowing to stand can be practiced by optionally selecting a time and temperature depending on the material of the container or the substrate, the kind of the base film forming agent for cell culture, and is practiced, for example, 1 minute to 24 hours, preferably 5 minutes to 3 hours at 10 to 80°C. According to the procedure, a cell culture container having the base film for cell culture at least part of the surface of the container, preferably over the entire surface, can be produced.

Also, the coating film on the surface of the container or the substrate obtained by such a method can be used as a cell culture container, after the step of contacting at least part of the surface of the above-mentioned container or substrate, preferably after the step of adding the composition for forming a coating film and allowing to stand for a predetermined time, without subjecting to a drying step and as such, or after washing using water or a medium (for example, water, buffer, culture medium, etc.) of the sample applied to cell culture.

That is, it can be used as a cell culture container after the step of contacting at least part of the surface of the above-mentioned container or substrate, preferably after the step of adding the composition for forming a coating film and allowing to stand for a predetermined time, within 48 hours, preferably within 24 hours, further preferably within 12 hours, further preferably within 6 hours, further preferably within 3 hours, further preferably within 1 hour without subjecting to the drying step and as such, or after washing with water or a medium (for example, water, buffer, culture medium, etc., particularly preferably medium (for example, DMEM medium (Dulbecco's modified Eagle's medium)) of a sample applied to cell culture.

The container may be applied to a drying step. The drying step is carried out under an atmosphere or vacuum, preferably at a temperature in the range of -200°C to 200°C. By the drying step, the solvent in the above-mentioned base film forming agent is removed, whereby it is completely adhered to the substrate.

The coating film can be formed by drying, for example, at room temperature (10°C to 35°C, preferably 20°C to 30°C, for example, 25°C), and may be dried, for example, at 40°C to 100°C to form a base film more rapidly. More preferable drying temperature is 10°C to 180°C, and more preferable drying temperature is 20°C to 150°C.

The coating film of the present application is produced by the simple and easy step as above.

Also, in order to eliminate impurities, unreacted monomers, etc., remained on the coating film, a step of washing with at least one kind of a solvent(s) selected from water and an aqueous solution containing an electrolyte(s) may be carried out. For washing, running water washing or ultrasonic wave washing, etc., is desirable. The above-mentioned water and aqueous solution containing an electrolyte(s) may be heated in the range of, for example, 40°C to 95°C. The aqueous solution containing an electrolyte(s) is preferably PBS, physiological saline (containing only sodium chloride), Dulbecco's phosphate buffered physiological saline, tris buffered physiological saline, HEPES buffered physiological saline and Veronal buffered physiological saline, and PBS is particularly preferable. After fixing, the coating film does not dissolve out and firmly fixed to the substrate even when it is washed with water, PBS and alcohol, etc.

A film thickness of the coating film of the present application is in the range of 1 to 1,000 nm, preferably in the range of 5 to 500 nm, 10 to 300 nm, 10 to 200 nm, 10 to 100 nm and 10 to 50 nm.

### <Laminated film>

The laminated film of the present application is a laminated film having a layer of the above-mentioned coating film and a layer of the base film for cell culture on the upper surface thereof.

The above-mentioned base film for cell culture may be a laminated film containing a copolymer (P²) which contains a recurring unit derived from a monomer represented by the following formula (I): 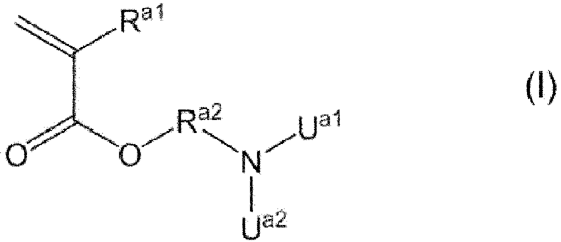 [wherein
U^{a1} and U^{a2} each independently represent a hydrogen atom or an alkyl group having 1 to 5 carbon atoms, R^{a1} represents a hydrogen atom or an alkyl group having 1 to 5 carbon atoms, and R^{a2} represents an alkylene group having 1 to 5 carbon atoms], and a recurring unit derived from a monomer represented by the following formula (II): 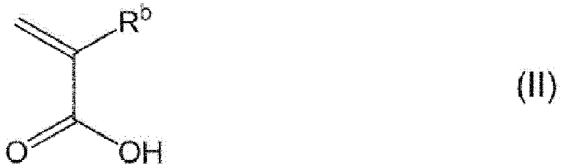 [wherein
R^{b} represents a hydrogen atom or an alkyl group having 1 to 5 carbon atoms] described in WO 2020/040247. Incidentally, examples of the above-mentioned "alkyl group having 1 to 5 carbon atoms" and "alkylene group having 1 to 5 carbon atoms" are the same as those mentioned in the explanation of the formula (1) and the formula (2).

The base film for cell culture of the present invention may be a material containing the base film for cell culture described in WO 2020/040247. The entire disclosure described in WO 2020/040247 is incorporated in the present application by reference.

A range of a maximum film thickness and a minimum film thickness of the above-mentioned base film for cell culture is, for example, in the range of 1 to 1,000 nm, and preferably in the range of 5 to 500 nm.

It may be a substrate for cell culture provided with the above-mentioned laminated film. Explanation regarding the above-mentioned substrate is as mentioned above.

### <Method for producing cell aggregates>

The method for producing a cell aggregate(s) of the present application includes a step of seeding cells onto the above-mentioned substrate for cell culture.

Production of the cell aggregate(s) can be carried out by a known method, and there may be mentioned static culture in which cells are cultured statically after seeding cells, and stirring culture (including shaking culture) in which cell suspension is stirred using a stirring blade after seeding cells, etc. In the stirring culture, there may be a time that the cell suspension stands still by stopping stirring for a predetermined time before and after, and during stirring, or stirring and standing may be repeated. For example, it can be produced by the method described in the following Examples. There is no limitation on the kind of the cells, and the above-mentioned cell can be mentioned as a specific example.

### EXAMPLES

Hereinafter, the present invention will be more specifically explained by referring to Examples and Comparative Examples, but the present invention is not limited to the following Examples.

The weight average molecular weight (Mw) of the polymer (A) shown in the following Synthetic Example is a measured result by a gel permeation chromatography (Gel Permeation Chromatography, GPC) method. The measurement conditions are as mentioned below.
GPC column: Plgel 3 µm MIXED-E (manufactured by Agilent Technologies)
Column temperature: 40°C
Solvent :Tetrahydrofuran (THF)
Flow rate: 1.00 mL/min
Detector: RI detector
Standard sample: Polystyrene

### [Synthesis of block copolymer]

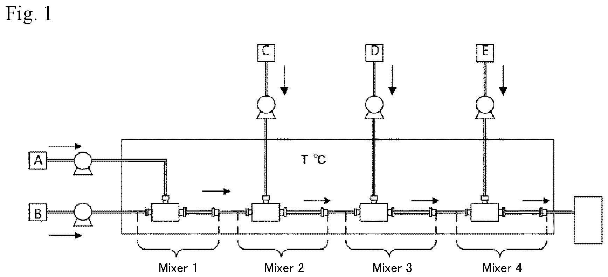

A schematic drawing of a flow reactor (reaction device) used in the following Synthetic Examples 1 to 4 and 6 is shown in Fig. 1. Incidentally, in Fig. 1, the arrow indicates the direction in which the liquid flows. A plunger pump A (manufactured by Flom, UI-22-110) was used for feeding the first monomer liquid, the plunger pump A and a mixer 1 were connected using a tube made of PTFE (inner size of 1.0 mm, outer size of 1.6 mm and length of 2 m), a syringe pump B (manufactured by YMC CO., LTD., Keychem-L) was used for feeding the initiator solution, and the syringe pump B and the mixer 1 were connected using a tube made of PTFE (inner size of 1.0 mm, outer size of 1.6 mm and length of 2 m). The outlet of the mixer 1 and the inlet of a mixer 2 were connected with the tube made of PFA (inner size of 2.0 mm, outer size of 3 mm and length of 1 m). At the other inlet of the mixer 2, a syringe pump C (manufactured by YMC CO., LTD., Keychem-L) for feeding a reaction adjusting agent solution was connected using the tube made of PEFE (inner size of 1.0 mm, outer size of 1.6 mm and length of 2 m) whereby the syringe pump C and the mixer 2 were connected. The outlet of the mixer 2 and the inlet of a mixer 3 were connected using a tube made of PFA (inner size of 2.0 mm, outer size of 3 mm and length of 1 m). At the other inlet of the mixer 3, using a syringe pump D (manufactured by YMC CO., LTD., Keychem-L) for feeding a second monomer solution and the tube made of PEFE (inner size of 1.0 mm, outer size of 1.6 mm and length of 2 m), the syringe pump D and the mixer 3 were connected. The outlet of the mixer 3 and the inlet of the mixer 4 were connected with the tube made of PFA (inner size of 2.0 mm, outer size of 3 mm and length of 5 m). At the other inlet of the mixer 4, using a plunger pump E (manufactured by Flom, UI-22-110) for feeding a polymerization terminator solution and a tube made of PEFE (inner size of 1.0 mm, outer size of 1.6 mm and length of 2 m), the plunger pump E and the mixer 4 were connected. At the outlet of the mixer 4, a tube made of PFA (inner size of 2.0 mm, outer size of 3 mm and length of 0.7 m) was connected. Incidentally, the flow path from the pumps A, B and C to the tip and the tube connected to the outlet of the mixer 2 up to 90% of the length was immersed in a water bath, and the flow path from the pumps D and E to the tip and the tube connected to the outlet of the mixer 4 up to 90% of the length was immersed in an outlet constant temperature bath at -40°C to adjust the temperature. As the mixer 1 used for the synthesis, Comet X-01 (made of stainless steel) manufactured by Techno Applications Co., Ltd., was used, as the mixers 2 and 3, a mixer for mixing two liquids [for the joint member and tubular body, those made of stainless were used, and as the static mixer element body, DSP-MXA3-17 (element made of polyacetal, number of twisted blades of 17, 3 mm diameter) manufactured by Noritake Co., Ltd., was processed] having a double-tube structure described in WO 2017/135398 was used, and as the mixer 4, a general simple double-tube mixer was used. Incidentally, as the connection method to each mixer, the first monomer solution tube was connected to the inlet of the introduction hole of the mixer 1, and the initiator solution tube was connected to the inlet of the inner tube. A tube connected to the outlet of the mixer 1 was connected to the inlet of the introduction hole of the mixer 2, and a reaction adjusting agent solution feeding tube was connected to the inlet of the inner tube. A tube connected to the outlet of the mixer 2 was connected to the inlet of the introduction hole of the mixer 3, and the second monomer solution tube was connected to the inlet of the inner tube. A polymerization inhibitor solution was connected to the inlet of the introduction hole of the mixer 4, and a tube connected to the outlet of the mixer 3 was connected to the inlet of the inner tube.

### <Synthetic Example 1: Synthesis of polystyrene-b-poly(diethylene glycol monomethyl ether methacrylate) block copolymer (PS-b-PDEGMA)>

0.25 mol/L styrene THF solution (containing 210 mg of lithium chloride as an additive salt) as the first monomer and 0.05 mol/L n-butyllithium toluene/hexane solution as an initiator were mixed with the mixer 1 at a flow rate of 10 mL/min and 2.0 mL/min, respectively, to polymerize the first monomer. Subsequently, 1,1-diphenylethylene (DPE) was mixed with the mixer 2 at 19 µL/min to adjust the reactivity with the second monomer. As the second monomer, diethylene glycol monomethyl ether methacrylate solution was mixed with the mixer 3 at 1.85 mL/min to carry out block polymerization. Subsequently, 0.25 mol/L methanol/THF solution as a polymerization terminator was mixed with a mixer 4 at 10 mL/min to terminate the polymerization. Each pump was fed for 3 minutes and the effluent was collected.

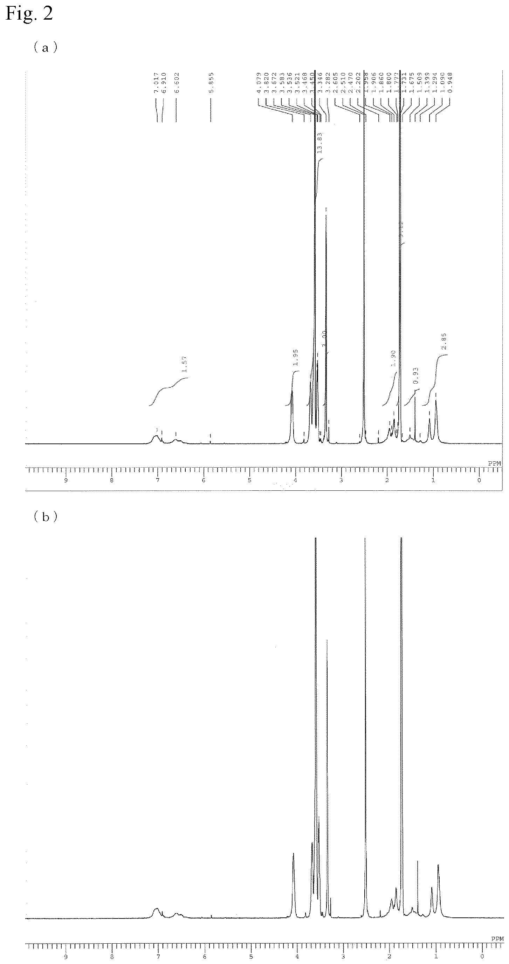

Further, the solvent of the above-mentioned effluent was almost distilled off by an evaporator and the residue was added dropwise to a stirred solution of 300 ml of diethyl ether, and the supernatant was removed from the obtained white suspension to obtain a precipitate. Subsequently, the obtained precipitate was vacuum dried to obtain 1.60 g of PS-b-PDEGMA. When the obtained copolymer was analyzed by GPC, it was Mn=41,258 and Mw/Mn=1.15. Also, from the results of ¹H-NMR of the copolymer, the composition ratio of the polystyrene unit and the diethylene glycol monomethyl ether methacrylate unit was polystyrene unit : diethylene glycol monomethyl ether methacrylate unit=24 : 76. ¹H-NMR chart of the obtained polymer is shown in Fig. 2.

### <Synthetic Example 2: Synthesis of polystyrene-b-poly(diethylene glycol monomethyl ether methacrylate) block copolymer (PS-b-PDEGMA)>

In the same manner as in Synthetic Example 1 except that the concentration of the initiator solution was made 0.09 mol/L, DPE was mixed with the mixer 2 at 35 µL/min, and a diethylene glycol monomethyl ether methacrylate solution was mixed as the second monomer the mixer 3 at 2.77 mL/min, synthesis was carried out to obtain 3.29 g of PS-b-PDEGMA. When the obtained copolymer was analyzed by GPC, it was Mn=24,751 and Mw/Mn=1.39. Also, from the results of ¹H-NMR of the copolymer, the composition ratio of the polystyrene unit and the diethylene glycol monomethyl ether methacrylate unit was polystyrene unit : diethylene glycol monomethyl ether methacrylate unit=11 : 89.

### <Synthetic Example 3: Synthesis of polystyrene -b-poly(diethylene glycol monomethyl ether methacrylate) block copolymer (PS-b-PDEGMA)>

In the same manner as in Synthetic Example 1 except that the solution concentration of the first monomer was made 0.20 mol/L (containing 395 mg of lithium chloride as an additive salt), the concentration of the initiator solution was made 0.09 mol/L, DPE was mixed with the mixer 2 at 35 µL/min, a diethylene glycol monomethyl ether methacrylate solution was mixed as the second monomer with the mixer 3 at 4.06 mL/min, and each pump was fed for 1 minute, synthesis was carried out to obtain 1.50 g of PS-b-PDEGMA. When the obtained copolymer was analyzed by GPC, it was Mn=21,721 and Mw/Mn=1.50. Also, from the results of ¹H-NMR of the copolymer, the composition ratio of the polystyrene unit and the diethylene glycol monomethyl ether methacrylate unit was polystyrene unit: diethylene glycol monomethyl ether methacrylate unit=8 : 92.

### <Synthetic Example 4: Synthesis of polystyrene -b-poly(diethylene glycol monomethyl ether methacrylate) block copolymer (PS-b-PDEGMA)>

In the same manner as in Synthetic Example 1 except that the solution concentration of the first monomer was made 0.10 mol/L (containing 356 mg of lithium chloride as an additive salt), the concentration of the initiator solution was made 0.09 mol/L, DPE was mixed with the mixer 2 at 35 µL/min and a diethylene glycol monomethyl ether methacrylate solution was mixed as the second monomer with the mixer 3 at 4.06 mL/min, synthesis was carried out to obtain 4.70 g of PS-b-PDEGMA. When the obtained polymer was analyzed by GPC, it was Mn=32,175 and Mw/Mn=1.28. Also, from the results of ¹H-NMR of the polymer, the composition ratio of the polystyrene unit and the diethylene glycol monomethyl ether methacrylate unit was polystyrene unit : diethylene glycol monomethyl ether methacrylate unit=3 : 97.

### <Synthetic Example 5: Synthesis of polystyrene -r-poly(diethylene glycol monomethyl ether methacrylate) random copolymer (PS-r-PDEGMA)>

To 55.3 mg of styrene and 2.0 g of diethylene glycol monomethyl ether methacrylate were added 18.50 g of toluene and 11.1 mg of 2,2'-azodiisobutyronitrile and dissolved. Thereafter, under nitrogen atmosphere, the temperature of the water bath was set to 75°C, and the mixture was stirred for 24 hours. After the temperature of the reaction solution was returned to room temperature, the solvent was almost distilled off by an evaporator and the residue was added dropwise to a stirred solution of 300 ml of diethyl ether, and the supernatant was removed from the obtained white suspension to obtain a precipitate. Further, the obtained precipitate was vacuum dried to obtain 0.64 g of PS-b-PDEGMA. When the obtained copolymer was analyzed by GPC, it was Mn=10,361 and Mw/Mn=2.26. Also, from the results of ¹H-NMR of the copolymer, the composition ratio of the polystyrene unit and the diethylene glycol monomethyl ether methacrylate unit was polystyrene unit : diethylene glycol monomethyl ether methacrylate unit=7 : 93.

### <Synthetic Example 6: Synthesis of polystyrene -b-poly(hydroxyethyl methacrylate) block copolymer (PS-b-PHEMA)>

In the same manner as in Synthetic Example 1 except that the solution concentration of the first monomer was made 0.10 mol/L (containing 117 mg of lithium chloride as an additive salt), the concentration of the initiator solution was made 0.05 mol/L, DPE was mixed with the mixer 2 at 17 µL/min and a 2-(trimethylsiloxy)ethyl methacrylate solution was mixed as the second monomer with the mixer 3 at 1.94 mL/min synthesis was carried out to obtain 1.25 g of PS-b-PHEMA precursor. When the obtained polymer was analyzed by GPC, it was Mn=28,877 and Mw/Mn=1.67. Subsequently, the obtained precursor polymer was suspended in 500 mL of diethyl ether and the suspension was added 500 µL of 35% hydrochloric acid and stirred overnight at room temperature. This suspension was filtered through a 1.0 µm membrane filter to obtain 1.08 g of PS-b-PHEMA. From the results of ¹H-NMR of the polymer, it was confirmed that the peak derived from trimethylsilyl protective group was completely disappeared. Also, the composition ratio of the polystyrene unit and the polyhydroxyethyl methacrylate unit was polystyrene unit : polyhydroxyethyl methacrylate unit=6.5 : 93.5.

### <Preparation Examples 1 to 5: preparation of polymer ethanol/aqueous solution>

The polymers of Synthetic Examples 1 to 5 were each dissolved in ethanol/water (95/5 weight percent concentration) so that the concentration became 10 mg/g to prepare compositions for forming a coating film (Preparation Examples 1 to 5).

### <Preparation Example 6: preparation of polymer ethanol solution>

The polymer of Synthetic Example 6 was dissolved in ethanol so that the concentration became 10 mg/g to prepare a composition for forming a coating film (Preparation Examples 6).

### <Comparative Preparation Examples 1: Preparation of polymer aqueous solution>

Polyethylene glycol 20000 (available from Junsei Chemical Co., Ltd., weight average molecular weight of 15,500 to 253,000) was dissolved in pure water so that the concentration became 10 mg/g to prepare a composition for forming a coating film (Comparative Preparation Examples 1).

### <Reference Example 1>

49.75 g of ethanol was added to 0.25 g of Lipidure-CM5206 (available from NOF CORPORATION) and sufficiently stirred to prepare a composition for forming a coating film (Reference Example 1).

### <Test Example 1: Coating film forming test>

The compositions for forming a coating film obtained in the above-mentioned Preparation Examples 1 to 6 and Comparative Preparation Example 1 were each spin coated onto a silicon wafer subjected to HMDS treatment at 1,500 rpm/60 sec, and as a drying step, it was dried in an oven at 50°C for 24 hours. Thereafter, each was thoroughly washed with PBS and then dried in an oven at 50°C for 1 hour to obtain a coating film on the silicon wafer subjected to HMDS treatment. The film thickness of the coating film on the silicon wafer subjected to HMDS treatment was measured using a spectroscopic ellipsometer and shown in the following Table 1. When any of the compositions for forming a coating film obtained in Preparation Examples 1 to 6 was used, the coating film was formed. When the composition for forming a coating film obtained in Comparative Preparation Example 1 was used, cissing of the film was caused on the substrate and a uniform film was not formed.

**[Table 1]**

| Coating agent | Film thickness [nm] after coating | Film thickness [nm] after immersion in PBS |
|---|---|---|
| Preparation Example 1 | 44. 6 | 44. 4 |
| Preparation Example 2 | 41. 7 | 36. 2 |
| Preparation Example 3 | 43. 0 | 35. 5 |
| Preparation Example 4 | 41.9 | 37. 8 |
| Preparation Example 5 | 44. 29 | 27. 8 |
| Preparation Example 6 | 42.41 | 41.94 |
| Comparative Preparation Example 1 | 2.12 | 1. 84 |
| None | 1. 8 | 1 8 |

### <Test Example 2: Colloid particle size measurement test>

When the compositions for forming a coating film obtained in the above-mentioned Preparation Examples 1, 2 and 4 were each placed in a glass vessel and exposed to the light of an LED light from the side, then the Tyndall phenomenon was observed so that it was confirmed that these were not a true solution but a colloidal dispersion. Next, with regard to the compositions for forming a coating film obtained in the above-mentioned Preparation Examples 1, 2 and 4, the average particle size was measured by a dynamic light scattering method (DLS) using Zetasizer Nano ZS (manufactured by Malvern Panalytical).

**[Table 2]**

| Coating agent | Primary particle size [nm] | Secondary particle size [nm] |
|---|---|---|
| Preparation Example 1 | 11. 2 | 236. 6 |
| Preparation Example 2 | 9. 0 | 172. 4 |
| Preparation Example 4 | 44. 0 | - |

### <Test Example 3: Protein adhesion suppressing test>

### (Preparation of coating plate)

The compositions for forming a coating film prepared in Preparation Examples 1 to 6 were each added to separate well of a 96-well cell culture plate (#9017, volume of 0.36 mL, made of polystyrene, manufactured by Corning) so as to be 20 µL/well per each 5 wells, and after the solvent was volatilized at room temperature, the cells were dried at 50°C using an oven for 24 hours. Thereafter, the coated each well was washed three times with 100 µL of pure water, dried using an oven at 50°C for 1 hour, and then used for the test. As a positive control, that in which the composition for forming a coating film prepared in Reference Example 1 was coated to a well of a 96-well cell culture plate (#9017, volume of 0.36 mL, made of polystyrene, manufactured by Corning) by the same method as mentioned above was used. As a negative control, a well of a 96-well cell culture plate (#9017, volume of 0.36 mL, made of polystyrene, manufactured by Corning) to which no coating had been applied was used.

### (Preparation of IgG-HRP diluted solution)

Goat anti-mouse IgG antibody HRP conjugate (available from Southern Biotechnology Associates) was diluted with PBS so as to have a concentration of 1 mg/g to prepare an IgG-HRP diluted solution.

### (Protein adhesion test)

100 µL/well of the IgG-HRP diluted solution was added to each well, positive control and negative control of the plate prepared as mentioned above, and the mixture was allowed to stand at room temperature for 30 minutes. After 30 minutes, the IgG-HRP diluted solution was discharged and each well was washed three times with 200 µL of PBS. TMB solution (available from sera care, SureBlue) was added to each well with 100 µL/well, and TMB STOP solution (available from sera care) was added after 1 minute with each 100 µL/well Using a microplate reader (infinite M200PRO manufactured by TECAN), absorbance at 450 nm and 650 nm was measured. The value obtained by subtracting the absorbance at 650 nm from the absorbance at 450 nm was calculated, and the average absorbance of 5 wells was obtained for each composition for forming a coating film. The average absorbance in the well of the negative control was made the protein adsorption rate of 100%, and the value calculating the protein adsorption ratio of the well to which each composition for forming a coating film had been coated was shown in Table 3. In either of the wells to which each composition for forming a coating film had been coated, the protein adhered amount was smaller than that in the ell (negative control) to which no coating had been applied.

**[Table 3]**

| Coating agent | Absorbance (450nm-650nm) | Protein adsorption ratio [%] |
|---|---|---|
| Preparation Example 1 | 0. 1178 | 20.0 |
| Preparation Example 2 | 0. 0615 | 10.4 |
| Preparation Example 3 | 0. 0208 | 3.52 |
| Preparation Example 4 | 0. 0298 | 5.05 |
| Preparation Example 5 | 0. 0229 | 3.88 |
| Preparation Example 6 | 0. 04208 | 7. 13 |
| Reference Example 1 | 0. 1181 | 20.01 |
| None | 0. 5901 | 100 |

### <Test Example 4: Cell adhesion suppressing test using mouse fibroblast>

### (Preparation of coating plate)

The compositions for forming a coating film prepared in Preparation Examples 1 to 6 were each added to separate well of the 96-well cell culture plate (#351172, volume of 0.37 mL, made of polystyrene, manufactured by Corning) so as to be 200 µL/well, and after allowing to stand at room temperature for 1 hour, excess varnish was removed. Using an oven, it was dried at 50°C for 24 hours. Thereafter, the coated each well was washed three times with 250 µL of pure water, dried using an oven at 50°C for 1 hour, and then used for the test. As a positive control, that in which the composition for forming a coating film prepared in Reference Example 1 was coated to a well of a 96-well cell culture plate (#351172, volume of 0.37 mL, made of polystyrene, manufactured by Corning) by the same method as mentioned above was used. As a negative control, a 96-well cell culture plate (#351172, volume of 0.37 mL, made of polystyrene, manufactured by Corning) to which no coating had been applied was used.

### (Preparation of cells)

As cells, mouse embryo fibroblasts C3H10T1/2 (available from DS Pharma Biomedical) were used. As the medium used for cell culture, BME medium (available from Thermo Fisher Scientific K. K.) containing 10%FBS (available from Sigma-Aldrich) and an L-glutamine-penicillin-streptomycin stabilized solution (available from Thermo Fisher Scientific K. K.) was used. The cells were statically cultured for 2 days or longer in a petri dish (medium 10 mL) having a diameter of 10 cm while maintaining a 5% carbon dioxide concentration in a 37°C/CO₂ incubator. Subsequently, after washing the present cells with 5 mL of PBS, 1 mL of 0.25 w/v% trypsin-1 mmol/L EDTA solution (available from FUJIFILM Wako Pure Chemical Corporation) was added thereto to peel off the cells, and the cells were each suspended in 10 mL of the above-mentioned medium. After the present suspension was centrifuged (model number: LC-200, 1,000 rpm/3 min, room temperature, manufactured by TOMY SEIKO Co., Ltd.), the supernatant was removed and the above-mentioned medium was added to prepare a cell suspension.

### (Cell adhesion test)

To each well, to the positive control and to the negative control of the plate prepared as mentioned above, each 150 µL of the cell suspension was added so as to be 1 x 10⁴ cells/well. Thereafter, while maintaining the 5% carbon dioxide concentration, it was allowed to stand in a CO₂ incubator at 37°C for 3 days.

### (Observation of cell adhesion)

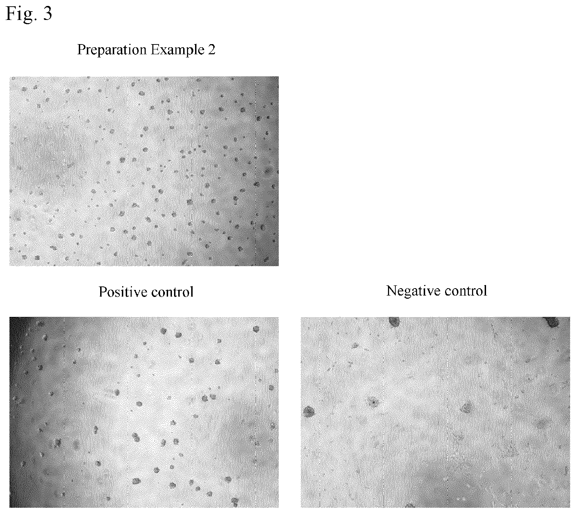

After 3 days of the culture, adhesion of cells to each well, to the positive control and to the negative control of the plate prepared as mentioned was compared with an inverted microscope (ECLIPSE TS100-F manufactured by Nikon Corporation). The results are shown in Table 4. As shown in Table 4, in the plates whose surfaces were treated with the compositions for forming a coating film prepared in Preparation Examples 1 to 6 and Reference Example 1 (positive control), cell adhesion and elongation were suppressed, and formation of cell aggregates (spheroids) in the well was observed. On the other hand, in the plate to which no coating was applied (negative control), although slight formation of spheroids was observed, most of the cells adhered to the bottom surface of the plate and elongated. Incidentally, as a representative example of cell observation, photomicrographs of each well of the plate coated with the compositions for forming a coating film of Preparation Example 2 and the positive control (Reference Example 1), and the negative control are shown in Fig. 3.

**[Table 4]**

| Coating agent | Cell adhesion |
|---|---|
| Preparation Example 1 | Slightly present |
| Preparation Example 2 | None |
| Preparation Example 3 | None |
| Preparation Example 4 | None |
| Preparation Example 5 | Slightly present |
| Preparation Example 6 | None |
| Positive control | None |
| Negative control | Present |

### <Test Example 5: Cell adhesion suppressing test using mesenchymal stem cells originated from human adipose tissue>

### (Preparation of coating plate)

Using the compositions for forming a coating film prepared in Preparation Examples 3 and 6, and Reference Example 1, a coating plate was prepared in the same manner as in Test Example 4.

### (Preparation of cells)

As the cells, mesenchymal stem cells originated from human adipose tissue ADSC (available from CellSource Co., Ltd.) were used. As the medium used for culturing the cells, a low serum medium Mesenchymal Stem Cell Growth Medium 2 medium (available from PromoCell) containing an L-glutamine-penicillin-streptomycin stabilized solution (available from Thermo Fisher Scientific K. K.) was used. The cells were statically cultured for 2 days or longer in a petri dish (medium 10 mL) having a diameter of 10 cm while maintaining a 5% carbon dioxide concentration in a 37°C/CO₂ incubator. Subsequently, after washing the present cells with 5 mL of PBS, 1 mL of TrypLE Select Enzyme (available from Thermo Fisher Scientific K. K.) was added thereto to peel off the cells, and the cells were each suspended in 10 mL of the above-mentioned medium. After the present suspension was centrifuged (model number: LC-200, 1,000 rpm/3 min, room temperature, manufactured by TOMY SEIKO Co., Ltd.), the supernatant was removed and the above-mentioned medium was added to prepare a cell suspension.

### (Cell adhesion test)

To each well, to the positive control and to the negative control of the plate prepared as mentioned above, each 150 µL of the cell suspension was added so as to be 1 x 10⁴ cells/well. Thereafter, while maintaining the 5% carbon dioxide concentration, it was allowed to stand in a CO₂ incubator at 37°C for 3 days.

### (Observation of cell adhesion)

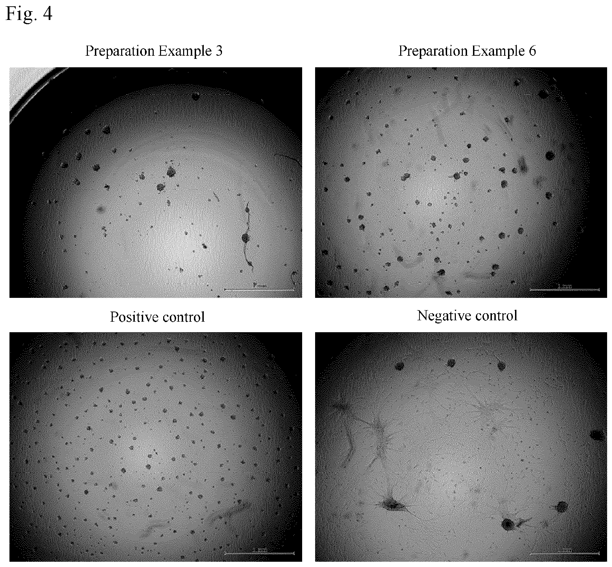

After 3 days of the culture, adhesion of cells to each well, to the positive control and to the negative control of the plate prepared as mentioned was compared with an inverted microscope (CKX31 manufactured by Olympus Corporation). The results are shown in Table 5. As shown in Table 5, in the plates whose surfaces were treated with the compositions for forming a coating film prepared in Preparation Examples 3 and 6 and Reference Example 1 (positive control), cell adhesion and elongation were suppressed, and formation of cell aggregates (spheroids) in the well was observed. On the other hand, in the plate to which no coating was applied (negative control), although slight formation of spheroids was observed, most of the cells adhered to the bottom surface of the plate and elongated. Photomicrographs of each well of the plate coated with the compositions for forming a coating film of Preparation Examples 3 and 6 and the positive control (Reference Example 1), and the negative control are shown in Fig. 4.

**[Table 5]**

| Coating agent | Cell adhesion |
|---|---|
| Preparation Example 3 | None |
| Preparation Example 6 | None |
| Positive control | None |
| Negative control | Present |

### <Test Example 6: Formation test of cell aggregates using mouse fibroblasts>

### (Preparation of cell-low adhesion plate)

The composition for forming a coating film prepared in Preparation Examples 3 was added to separate well of the 24-well cell culture plate (#351147, culture area of 2 cm², made of polystyrene, manufactured by Corning) so as to be 0.5 mL/well, and after allowing to stand at room temperature for 1 hour, excess varnish was removed. Using an oven, it was dried at 50°C for 24 hours to obtain a cell-low adhesion plate.

### (Preparation of forming agent of base film for cell culture)

In accordance with the producing method described in Synthetic Example 8 of WO 2020/040247, a polymer to be used as a forming agent of a base film for cell culture was obtained. The obtained polymer was dissolved in water to a concentration of 2,000, 1,000, 500 or 150 µg/mL to prepare a forming agent of a base film for cell culture.

### (Preparation of plate for forming cell aggregates by inkjet)

Using an inkjet device (model number: LaboJet-600 manufactured by Microjet Co., Ltd.) and inkjet head (model number: IJHBS-1000), the forming agent of the base film for cell culture prepared as mentioned above was coated to the cell-low adhesion plate prepared as mentioned above with a suitable amount. It was dried in an oven at 70°C for 24 hours to prepare plates (well Nos. 1 to 4) for forming cell aggregates.

### (Preparation of cells)

In the same manner as in Test Example 4, preparation of the cells was carried out.

### (Observation of cell adhesion)

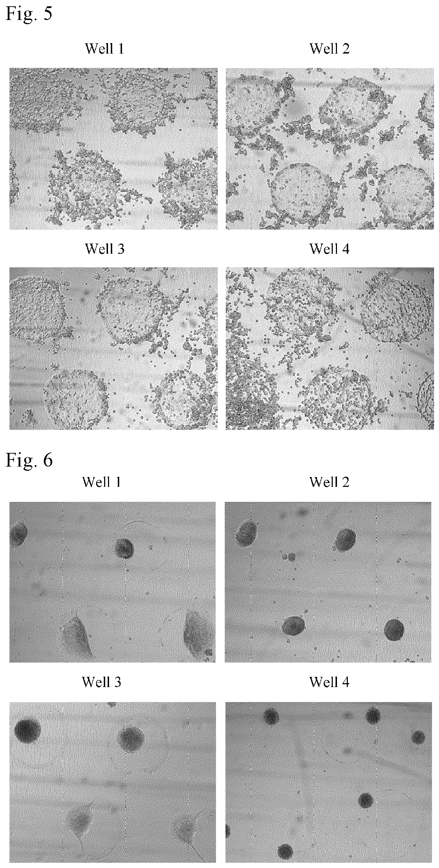

To the plate for forming cell aggregates prepared as mentioned was added each at 0.8 mL of the cell suspension so as to be 3.0×10⁵ cells/cm². Thereafter, it was allowed to stand in a CO₂ incubator at 37°C for 2 hours in the state of maintaining a 5% carbon dioxide concentration. After allowing to stand, the state of cell adhesion to the coated portion of each coating agent was confirmed using an inverted microscope (CKX31 manufactured by Olympus Corporation). As a results, as shown in Fig. 5, it was confirmed that the cells dispersed over the entire surface immediately after seeding selectively adhered to the part to which the forming agent of the base film for cell culture had been coated on the upper layer without adhering or elongating to the exposed parts of the composition for forming a coating film prepared in Preparation Examples 3 of the well Nos. 1 to 4.

### (Observation of cell aggregates)

The plate whose cell adhesion was confirmed as mentioned above was allowed to stand in a CO₂ incubator at 37°C for further 1 day. After allowing to stand, the state of the cells was observed using an inverted microscope (CKX31 manufactured by Olympus Corporation). As a results, as shown in Fig. 6, it was confirmed that the cells adhered to the part at which the forming agent of the base film for cell culture of the well Nos. 1 to 4 had been coated were peeled off from the plate and aggregated to form cell aggregates (spheroid). Also, at this time, no cells was adhered nor elongated at the part at which the composition for forming a coating film prepared in Preparation Examples 3 was exposed. From this fact, it was suggested that the base film containing the polymer of the present invention is useful as a cell low-adhesion film for cell culture containers. The results at each stage of maintenance of cell adhesion after 2 hours from seeding and formation of cell aggregates are shown in Table 6.

**[Table 6]**

| Well No. | Lower layer: Coating agent | Upper layer: Concentration [pg/mL] of forming agent of base film for cell culture | Cell adhesion after 2 hours | Cell aggregate formation |
|---|---|---|---|---|
| 1 | Preparation Example 3 | 2000 | ○ | ○ |
| 2 | Preparation Example 3 | 1000 | ○ | ○ |
| 3 | Preparation Example 3 | 500 | ○ | ○ |
| 4 | preparation Example 3 | 150 | ○ | ○ |

### <Test Example 7: Formation test of cell aggregates using mouse fibroblasts>

### (Preparation of cell low adhesion plate)

The composition for forming a coating film prepared in Preparation Examples 3 was spin coated on a PS sheet at 1,500 rpm/60 sec, and as a drying step, it was dried in an oven at 50°C for 24 hours. Thereafter, it was adhered to a 24-well type bottomless type plate to obtain a cell low adhesion plate

### (Preparation of forming agent of base film for cell culture)

In the same manner as in Test Example 5, a polymer to be used as a forming agent of the base film for cell culture was obtained. The obtained polymer was dissolved in water to a concentration of 2,000, 1,000 or 500 µg/mL to prepare a forming agent of a base film for cell culture.

### (Preparation of plate for forming cell aggregates by inkjet)

Using inkjet device (model number: LaboJet-600 manufactured by Microjet Co., Ltd.), and inkjet head (model number: 500-S-C), the forming agent of the base film for cell culture prepared as mentioned above was coated to the cell-low adhesion plate prepared as mentioned above with a suitable amount. It was dried in an oven at 70°C for 24 hours to prepare plates (well Nos. 5 to 7) for forming cell aggregates.

### (Preparation of cells)

In the same manner as in Test Example 4, preparation of cells was carried out.

### (Observation of cell adhesion)

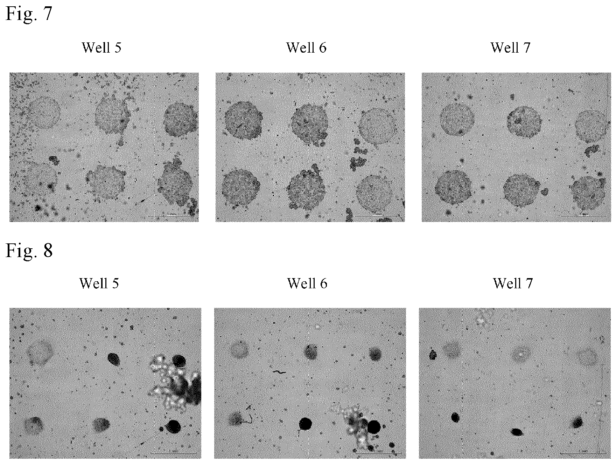

To the plate for forming cell aggregates prepared as mentioned was added each at 0.8 mL of the cell suspension so as to be 3.0x 10⁵ cells/cm². Thereafter, it was allowed to stand in a CO₂ incubator at 37°C for 2 hours in the state of maintaining a 5% carbon dioxide concentration. After allowing to stand, the state of cell adhesion to the coated portion of each coating agent was confirmed using an inverted microscope (CKX31 manufactured by Olympus Corporation). Thereafter, floating cells and the medium were removed by an aspirator, and washed with PBS to leave only adherent cells on the wells. After washing, each 0.8 mL of new medium was added, and the state of cell adhesion was observed using an inverted microscope (CKX31 manufactured by Olympus Corporation). As a results, as shown in Fig. 7, it was confirmed that the cells dispersed over the entire surface immediately after seeding selectively adhered to the part to which the forming agent of the base film for cell culture had been coated on the upper layer without adhering or elongating to the exposed parts of the composition for forming a coating film prepared in Preparation Examples 3 of the well Nos. 5 to 7, and adhesion of the cells was maintained even after the medium was replaced.

### (Observation of cell aggregates)

The plate whose cell adhesion was confirmed as mentioned above was allowed to stand in a CO₂ incubator at 37°C for further 1 day. After allowing to stand, the state of the cells was observed using an inverted microscope (CKX31 manufactured by Olympus Corporation). As a results, as shown in Fig. 8, it was confirmed that the cells adhered to the part at which the forming agent of the base film for cell culture of the well Nos. 5 to 7 had been coated were peeled off from the plate and aggregated to form cell aggregates (spheroid). Also, at this time, no cells was adhered nor elongated at the part at which the composition for forming a coating film prepared in Preparation Examples 3 was exposed. From this fact, it was suggested that the base film containing the polymer of the present invention is useful as a cell low-adhesion film for cell culture containers. The results at each stage of cell adhesion before washing, maintenance of cell adhesion after washing and formation of cell aggregates are shown in Table 7.

**[Table 7]**

| Well No. | Lower layer: Coating agent | Upper layer: Concentration [pg/mL] of forming agent of base film for cell culture | Cell adhesion before washing | Cell adhesion after washing | Cell aggregate formation |
|---|---|---|---|---|---|
| 5 | Preparation Example 3 | 2000 | ○ | ○ | ○ |
| 6 | Preparation Example 3 | 1000 | ○ | ○ | ○ |
| 7 | Preparation Example 3 | 500 | ○ | ○ | ○ |

### UTILIZABILITY IN INDUSTRY

According to the present invention, it is possible to provide a composition for forming a coating film having compatibility with a biological substance, a coating film using the same, and a substrate for cell culture using the same.

## Claims

1. A composition for forming a coating film which comprises
a copolymer (P¹) containing unit structures represented by the formula (1) and the formula (2): 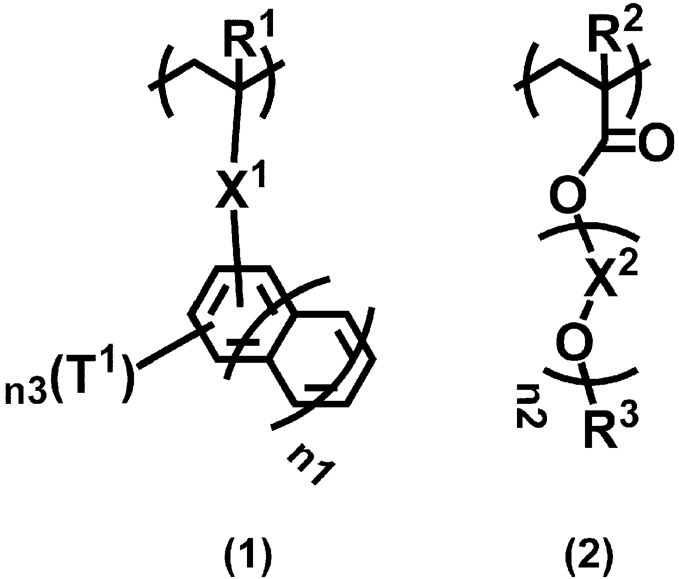
wherein R¹ to R³ each independently represent a hydrogen atom or an alkyl group having 1 to 5 carbon atoms, X¹ represents a single bond, an ester bond, an amide bond or an alkylene group having 1 to 5 carbon atoms, X² represents an alkylene group having 1 to 5 carbon atoms, T¹ represents a halogen atom, an alkyl group having 1 to 5 carbon atoms which may be substituted by a halogen atom(s), a hydroxyl group, a carboxyl group, a nitro group, a cyano group, a methylenedioxy group, an acetoxy group, a methylthio group, an amino group or an alkoxy group having 1 to 10 carbon atoms, n1 represents an integer of 0 to 2, n2 represents an integer of 1 to 10 and n3 represents an integer of 0 to 8,
but not containing a polysiloxane skeleton, and
a solvent.

2. The composition according to Claim 1, wherein the solvent is selected from water, an alcohol or a combination thereof.

3. The composition for forming a coating film according to Claim 1 or 2, which is in a form of a colloidal solution containing the solvent as a dispersion medium, and containing the copolymer (P¹) as a dispersoid.

4. The composition for forming a coating film according to any one of Claims 1 to 3, which has an ability to suppress adhesion of biological substances.

5. A coating film which comprises
a copolymer (P¹) containing unit structures represented by the formula (1) and the formula (2): 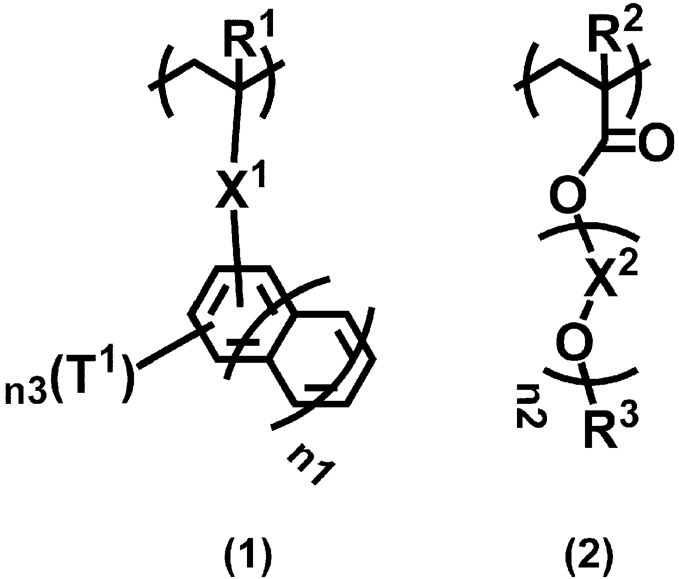
wherein R¹ to R³ each independently represent a hydrogen atom or an alkyl group having 1 to 5 carbon atoms, X¹ represents a single bond, an ester bond, an amide bond or an alkylene group having 1 to 5 carbon atoms, X² represents an alkylene group having 1 to 5 carbon atoms, T¹ represents a halogen atom, an alkyl group having 1 to 5 carbon atoms which may be substituted by a halogen atom(s), a hydroxyl group, a carboxyl group, a nitro group, a cyano group, a methylenedioxy group, an acetoxy group, a methylthio group, an amino group or an alkoxy group having 1 to 10 carbon atoms, n1 represents an integer of 0 to 2, n2 represents an integer of 1 to 10 and n3 represents an integer of 0 to 8,
but not containing a polysiloxane skeleton.

6. The coating film according to Claim 5, which has an ability to suppress adhesion of biological substances.

7. A substrate for cell culture which comprises the coating film according to Claim 6 onto at least part of a surface of the substrate.

8. A laminated film which comprises a layer of the coating film according to Claim 5 or 6, and a layer of a base film for cell culture on an upper surface thereof.

9. The laminated film according to Claim 8, wherein
the base film for cell culture contains a copolymer (P²) which contains a recurring unit derived from a monomer represented by the following formula (I): 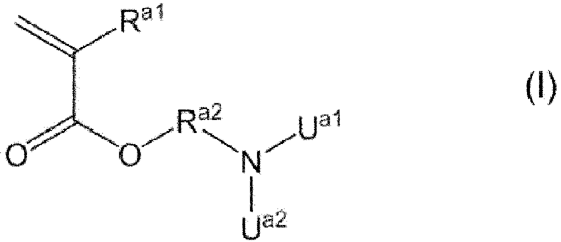 wherein
U^{a1} and U^{a2} each independently represent a hydrogen atom or an alkyl group having 1 to 5 carbon atoms, R^{a1} represents a hydrogen atom or an alkyl group having 1 to 5 carbon atoms, and R^{a2} represents an alkylene group having 1 to 5 carbon atoms, and a recurring unit derived from a monomer represented by the following formula (II): 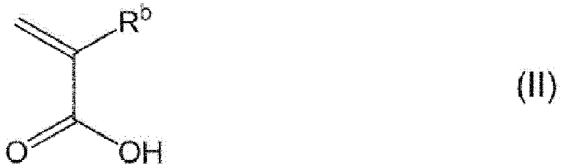 wherein
R^{b} represents a hydrogen atom or an alkyl group having 1 to 5 carbon atoms.

10. A substrate for cell culture provided with the laminated film according to Claim 8 or 9.

11. A method for producing a cell aggregate(s), which comprises a step of seeding cells to the substrate for cell culture according to Claim 7 or 10.
